# FASCICULE DE BREVET EUROPEEN

(11) **EP 0 876 312 B1**
(45) Date de publication et mention de la délivrance du brevet: **14.03.2001**
(21) Numéro de dépôt: 96941103.2
(22) Date de dépôt: 03.12.1996
(51) Int. Cl.: C07B 45/06, C07C 319/24, C07C 321/14

(54) **PROCEDE DE PREPARATION DE DISULFURES ET DE POLYSULFURES ORGANIQUES**
VERFAHREN ZUR HERSTELLUNG VON ORGANISCHEN DISULFIDEN UND POLYSULFIDEN
METHOD FOR PREPARING ORGANIC DISULPHIDES AND POLYSULPHIDES

(30) Priorité: 11.12.1995 FR 9514581
(43) Date de publication de la demande: 11.11.1998
(73) Titulaire: ELF AQUITAINE PRODUCTION, 92400 Courbevoie (FR)
(72) Inventeur: ARRETZ, Emmanuel, F-64000 Pau (FR)
(74) Mandataire: Leboulenger, Jean
(86) Numéro de dépôt international: FR9601921
(87) Numéro de publication internationale: WO9721649

(56) Documents cités:
- EP-A- 0 337 837
- FR-A- 1 527 462
- FR-A- 1 553 249
- US-A- 2 237 625

## Description

La présente invention concerne la production de disulfures et de polysulfures organiques R-Sₙ-R (avec n ≥ 2) par réaction de mercaptans avec le soufre en présence de résines basiques agissant comme catalyseurs selon la réaction :

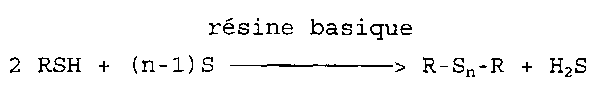

En présence de ces mêmes résines basiques, les disulfures et les polysulfures organiques de faible rang en soufre peuvent être transformés en polysulfures de rang supérieur en soufre par réaction avec le soufre. De même, en présence de ces mêmes résines basiques, les polysulfures organiques de rang élevé en soufre peuvent être transformés en polysulfures de rang inférieur en soufre par réaction avec des mercaptans.

Ainsi la demande de brevet EP-A-337837 enseigne la préparation de disulfures et de polysulfures organiques en présence de résines organiques échangeuses d'anions comportant des groupes fonctionnels amine tertiaire ou ammonium quaternaire (actives sous forme hydroxyde). De telles résines, généralement sous la forme de grains ou de billes insolubles dans les milieux réactionnels liquides et donc facilement séparables en fin de réaction, permettent d'obtenir des disulfures organiques et des polysulfures par réaction du soufre élémentaire avec des mercaptans et également d'obtenir des polysulfures organiques à rang élevé en soufre par réaction du soufre élémentaire sur des polysulfures organiques de rang inférieur en soufre.

S.V. Luis, M.I. Burguete et B. Altava, Reactive & Functional Polymers, 26, 1995, 75-83, indiquent que la chlorométhylation facile des résines poly-styréniques et la haute réactivité des sites benzyliques résultants permet l'introduction d'un grand nombre de groupes fonctionnels et explique l'usage étendu de ces polymères.

Par contre, ces auteurs remarquent que la longueur réduite du bras espaceur méthylène réduit la mobilité des groupes fonctionnels introduits et, dans certains cas, rend leur accessibilité difficile aux réactifs, substrats et solvants. Cette situation peut conduire à une diminution de l'activité des groupes fonctionnels lorsqu'on les compare à leurs correspondants solubles. Dans certains cas, une amélioration nette de l'activité de ces groupes liés à la résine a été obtenue lorsque le site actif est séparé du squelette polymérique par un bras espaceur approprié.

S.V. Luis et al. préparent des résines polystyréniques ayant des bras espaceurs sous forme de chaîne aliphatique linéaire comportant 6 ou 9 groupes méthylène et portant en bout de chaîne un groupe hydroxyle -OH.

Ce groupe hydroxyle est transformé en groupe partant tosylate, ce dernier étant remplacé par substitution par un groupe amine tertiaire.

Dans cette synthèse S.V. Luis et al. utilisent la fonctionnalisation de la résine polystyrénique par une réaction type Friedel Crafts à l'aide du chlorure d'acide dérivé d'un ester monoalkyle d'acide alcanedioïque.

Cette synthèse présente l'inconvénient majeur de la réduction à la fois d'un groupe tosylhydrazone et d'un groupe esters par de l'hydrure double LiAlH₄ dans le tétrahydrofuranne (THF). Cette réduction rend cette voie de synthèse peu attractive sur le plan d'un développement industriel en des quantités importantes de résines comportant ces bras espaceurs en C₆ ou C₉.

D'autres auteurs se sont intéressés à l'obtention de bras espaceurs sous la forme d'une chaîne méthylène. Ainsi M. Tomoi, N. Kori et H. Kakiuchi, Reactive Polymers, 3, 1985, 341-349, introduisent sur des résines polystyréniques une longue chaîne aliphatique par alkylation à l'aide d'ω-bromoalkènes en présence d'acide trifluorométhanesulfonique.

Cependant cette synthèse est limitée à la préparation de polymères à bras espaceur, présentant un faible degré de réticulation (0-4 %).

De leur côté, G.D.Darling et M.J. Fréchet, J. Org. Chem., 51, 1986, 2270-2276, en partant d'une résine polystyrénique chlorométhylée ont obtenu un bras espaceur -(CH₂)₂- séparant la résine d'un hydroxyle -OH en bout de chaîne. Cet hydroxyle est transformé en tosylate, puis par la réaction de Gabriel, à l'aide de phtalimide de potassium et enfin par de d'hydrazine, en amine primaire. Cette synthèse a cependant l'inconvénient d'utiliser du n-butyllithium ou de l'hydrure double de lithium et d'aluminium.

L'objet de la présente invention est de proposer un procédé de préparation de disulfures et polysulfures organiques, selon les réactions exposées ci-dessus, en présence de résines PS-DVB fonctionnalisées et spécialement sélectionnées ou synthétisées afin d'obtenir de meilleurs résultats que dans l'art antérieur. Ces meilleurs résultats peuvent être, par exemple, un meilleur taux de conversion des réactifs et/ou une cinétique de réaction plus rapide.

Ce but est atteint par la mise en oeuvre de résines fonctionnalisées par des groupes amine primaire -NH₂.

Plus précisément, la présente invention propose un procédé de préparation de disulfures et polysulfures organiques par réaction du soufre sur un mercaptan ou sur un polysulfure de rang inférieur en soufre pour le transformer en polysulfure de rang supérieur, ou encore par réaction d'un mercaptan sur un polysulfure organique de rang élevé en soufre pour le transformer en polysulfure de rang inférieur en soufre, en présence d'un catalyseur sous forme de résine à fonction basique, caractérisé en ce que la résine est à base de polystyrène-divinylbenzène (PS-DVB) fonctionnalisée par des groupes amine primaire et ayant la formule générale (I) : 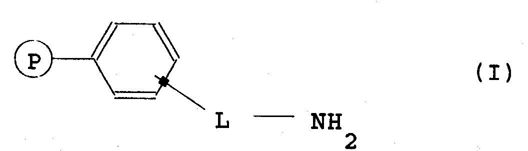 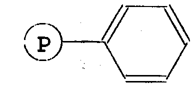 étant le support résine PS-DVB,
L étant un radical organique linéaire ayant une longueur égale ou supérieure à celle du radical méthylène (-CH₂-).

Les résines qui servent de matériaux de base pour la préparation des résines à fonction amine primaire de formule générale (I) peuvent être des copolymères de PS-DVB ou de copolymères PS-DVB chlorométhylés, qui, par des réactions chimiques appropriées qui seront décrites ci-après, sont transformés en résines à fonction -NH₂.

Avec un faible taux de divinylbenzène comme agent de réticulation, on obtient des copolymères du type gel, tandis qu'avec des teneurs plus élevées en DVB, on peut obtenir des résines macroréticulées et de structure macroporeuse.

Le taux de DVB peut être de 0,5 % à 60 % en poids par rapport au poids total du copolymère PS-DVB.

De préférence, les matériaux de base et, par voie de conséquence, les résines de formule générale (I) sont macroréticulées et de structure macroporeuse, car ces caractéristiques entraînent une meilleure activité catalytique dans le procédé que les résines de type gel.

Ces résines PS-DVB peuvent être chlorométhylées par l'éther chlorométhylique, selon des techniques connues et décrites dans la littérature, à des teneurs variables en chlore (Cl), généralement de 1 à 20 % en poids de chlore par rapport au poids de résine chlorométhylée.

De préférence, le radical L représente un méthylène. En effet, ces résines avec des groupes aminométhyle sont soit commerciales, soit d'une synthèse aisée.

Avantageusement, le radical L a la formule générale(II) suivante :

-CH₂-(-X-CH₂-CH₂-)ₘ- (II),

dans laquelle X représente l'oxygène ou le soufre et m vaut 1 ou 2.

De préférence, dans la formule (II), X est l'oxygène et m vaut 1, ou encore X est le soufre et m vaut 1.

Les résines PS-DVB à fonction amine primaire de formule générale (I) peuvent être obtenues par différentes techniques :
1/ on peut par exemple partir d'une résine de formule générale (B) : 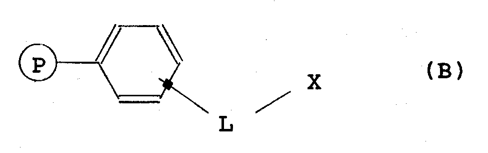 X étant un groupe partant notamment halogène ou tosylate obtenable à partir d'un groupe hydroxyle -OH, et L ayant la même signification que ci-dessus.
   De préférence, lorsque L représente un seul méthylène, X est un atome de chlore. Dans ce cas, une méthode, décrite par D.H. Rich et S.K. Gurwara, J. Am. Chem. Soc., 1975, 97 - 1575-1579, consiste à faire réagir une résine PS-DVB chlorométhylée avec un excès d'ammoniac. Une autre voie est basée sur l'obtention de résine PS-DVB phtalimidométhylée qui est transformée par hydrazinolyse en résine à fonction amine primaire. Les deux méthodes d'accès à de telles résines phtalimidométhylées sont décrites dans la publication de A.R. Mitchell, S.B.H. Kent, B.W. Erickson et R.B. Merrifield, Tetrahedron Letters N° 42, 1976, 3795-3798. L'une consiste à partir d'une résine PS-DVB qui par réaction avec le N-(chlorométhyl) phtalimide est directement convertie en résine phtalimidométhylée. L'autre méthode part d'une résine PS-DVB chlorométhylée qui est traitée par le phtalimide de potassium pour donner la résine correspondante phtalimidométhylée.
   Quelques résines PS-DVB à fonction amine primaire de formule (I) dans laquelle L représente un méthylène sont commerciales.
   Ainsi la Société PUROLITE propose deux résines macroporeuses l' A-107 et l' A-109, tandis que la Société FLUKA a, dans son catalogue 1994-1995, deux résines gels : la résine 08564 PS réticulée avec 2 % de DVB et contenant 1,1 mmole de groupe -NH₂ par gramme de résine, et la résine 08566 PS réticulée avec 1 % de DVB et contenant 0,6 mmole de -NH₂ par gramme de résine.
   La méthode au phtalimide de potassium est également applicable aux résines de formule (B) dans le cas où L est un radical organique linéaire d'une longueur supérieure à celle du radical méthylène, notamment -(CH₂)ₙ-, avec n valant un nombre supérieur à 1.
2/ on peut également partir d'une résine PS-DVB de formule (B) dans laquelle L représente un méthylène et X a la signification ci-dessus et de préférence représente un atome de chlore. La Demanderesse a trouvé que cette résine chlorométhylée peut être mise en réaction avec une alkanolamine ou une mercaptoalkylamine, sous forme d'alcoolate alcalin, dans les conditions de la réaction de Williamson.

Si on met en oeuvre l'éthanolamine, on obtient des résines PS-DVB à fonction amine primaire avec des groupes fonctionnels -CH₂-O-CH₂-CH₂-NH₂ fixés aux supports résines PS-DVB.

De manière analogue, en partant du 2-amino éthanethiol hydrochlorure, on obtient les groupes fonctionnels -CH₂-S-CH₂-CH₂-NH₂.

Si on utilise le 2-(2-aminoéthoxy)éthanol, on obtient des résines PS-DVB à fonction amine primaire avec des groupes fonctionnels -CH₂(-O-CH₂-CH₂)₂-NH₂.

Enfin, en utilisant le 2-(2-aminoéthyl)thioéthanethiol, on obtient des groupes fonctionnels -CH₂-(S-CH₂-CH₂)₂-NH₂.

Cette mercaptoalkylamine de départ peut être préparée selon Iwakura et al., J. Polym. Sci. Part A, 2, 1964, 881-883 ou selon I Voronkov, M.G. et al., Chem. Heterocycl. Compd. (Engl. Transl.)15, 1979, 1183-1185.

Les conditions générales de la réaction de Williamson sont les suivantes :

L'alcanolamine ou la mercaptoalkylamine diluée dans du tétrahydrofuranne (THF) anhydre, est mise à réagir avec de l'hydrure de sodium en suspension dans du THF anhydre. Après formation de l'alcanolate de sodium ou le mercaptide de sodium, la résine chlorométhylée est introduite dans le milieu réactionnel liquide.

Avantageusement, les disulfures, polysulfures organiques et mercaptans ont des radicaux hydrocarbonés R choisis parmi un groupe alkyle, cycloalkyle, aryle, aralkyle et alkylaryle.

La présente invention s'applique notamment à la production des disulfures et polysulfures de dialkyle contenant au total de 2 à 40 atomes de carbone, par exemple le disulfure et les polysulfures de diméthyle, diéthyle, dipropyle, dibutyle, dipentyle, dihexyle, diheptyle, dioctyle, didécyle et didodécyle. Elle s'applique également à la préparation de disulfures et polysulfures cycloalkyliques, par exemple, disulfure ou polysulfures de dicyclohexyle ou à la préparation par exemple de disulfure ou polysulfures de diphényle.

Avantageusement le radical hydrocarboné R porte un ou plusieurs groupes fonctionnels. Ces groupes sont, par exemple, les atomes d'halogène, -OH, -OR', -SR', NR'R'', CN, -CHO, -COR', -COOR', R' et R'' désignent des radicaux aliphatiques de C₁ à C₁₂, cycloaliphatiques, aromatiques ou alkylaromatiques.

L'activité catalytique des résines mises en oeuvre dans la présente invention, se manifeste à des teneurs très faibles de résines dans les mélanges.

Avantageusement, la résine est présente en une quantité allant de 0,01 à 20 parties en poids pour 100 parties en poids de mélange réactionnel, résine comprise.

Le procédé selon l'invention met en oeuvre une réaction qui peut être effectuée à une température de -10°C à 150°C. De préférence, la température est de +10°C à 120°C.

Les réactions peuvent être conduites à pression atmosphérique ou à des pressions supérieures pouvant atteindre 50 bars. En général, cette pression est de 28 bars absolu. Dans le cas de réactifs peu volatils et à faible tension de vapeur, la réaction peut être réalisée à des pressions inférieures à la pression atmosphérique, avec éventuellement la présence d'un gaz inerte, tel que l'azote.

Le rapport molaire mercaptan/soufre dépend de la nature du mercaptan utilisé et du produit à préparer (disulfure ou polysulfure). Avantageusement ce rapport est de 0,3 à 10 et de préférence de 0,4 à 6.

Dans le cas où l'on part d'un polysulfure organique de rang élevé en soufre que l'on veut transformer en polysulfure organique de faible rang en soufre, par exemple en trisulfure R-S₃-R ou disulfure R-S₂-R par action du mercaptan correspondant, avantageusement, on utilise un rapport molaire mercaptan/polysulfure allant de 2 à 10.

La production de disulfures ou de polysulfures organiques en présence des résines PS-DVB à fonction amine primaire peut être mise en oeuvre dans un réacteur agité ou tubulaire, suivant un procédé discontinu, soit par chargement des réactifs avant leur mise en réaction, soit par addition progressive de l'un des réactifs, soit, par addition simultanée des réactifs dans le réacteur, ou encore suivant un procédé continu avec addition contrôlée des réactifs.

Dans le cas où le soufre est l'un des réactifs (l'autre étant un mercaptan ou un polysulfure de faible rang en soufre), il peut être introduit sous la forme liquide ou solide.

En plus de la description qui précède, la présente invention sera mieux comprise à l'aide de la partie expérimentale qui suit à titre illustratif.

### Partie expérimentale

Les séchages des résines sont pratiqués sous un vide d'environ 4x10³ Pascal.

### A) Les résines mises en oeuvre :

La Société Purolite commercialise deux résines PS-DVB à fonction -CH₂-NH₂, macroréticulées et de structure macroporeuse :
- Résine A-107 : 4,1 méq de -NH₂/g de résine sèche.
- Résine A-109 : 4,3 méq de -NH₂/g de résine sèche.

La Société Rohm and Haas commercialise une résine PS-DVB à fonction -CH₂-N(CH₃)₂ macroréticulée et à structure macroporeuse :
- Résine Amberlyst A-21 : 4,4 méq de -NH₂/g de résine sèche.

D'autres résines ont été spécialement synthétisées et forment les exemples suivants :

### Exemple 1 Obtention d'une première résine de formule (I) dans laquelle L représente -CH₂-.

(a) La résine PS-DVB utilisée est un copolymère synthétique poreux commercialisé par la Société Rohm et Haas : l'Amberlite XAD-4.
Les caractéristiques de cette résine macroporeuse à surface spécifique élevée, fortement réticulée, sont d'après la fiche technique de Rohm et Haas :

| | |
|---|---|
| Surface spécifique | 750 m²/g |
| Diamètre moyen de pore | 50 Å |
| Volume des pores | 51% |

(b) Fonctionnalisation de cette résine par le N-(chlorométhyl)phtalimide
On ajoute 10 g de résine Amberlite XAD-4 préalablement séchée à une solution composée de 0,5 ml (0,0043 mole) de tétrachlorure d'étain dans 30 ml de 1,2-dichloroéthane, puis sous agitation et à une température de 60°C, on ajoute une solution de 6,7 g (0,0342 mole) de N-(chlorométhyl)phtalimide dans 20 ml de 1,2-dichloroéthane. Le milieu réactionnel est maintenu agité et au reflux (82-84 °C) pendant 5 heures. Après retour à la température ambiante, la résine est filtrée, lavée au 1,2-dichloroéthane, puis au méthanol. Après séchage sous vide à 60°C, on a obtenu 13,1 g de résine modifiée.
Spectre IR : bande ν et δ de CO-N-CO à 1770 cm⁻¹ et 1710 cm⁻¹.
(c) Formation de l'amine primaire par hydrazinolyse.
12 g de la résine modifiée obtenue sont mis dans 40 ml d'éthanol absolu. A cette suspension on ajoute 4,5 ml (0,092 mole) d'hydrate d'hydrazine et 0,9 g (0,022 mole) de soude en pastilles. L'ensemble est porté au reflux durant 48 heures. Après retour à la température ambiante la résine est filtrée, lavée à l'éthanol, puis traitée par une solution aqueuse de potasse à 5% en poids. La résine est ensuite lavée à l'eau jusqu'à pH neutre, à l'éthanol, à l'acétone et au méthanol. Après séchage sous vide, on obtient 11 g de résine.
- Spectre IR :: plus de bandes caractéristiques à 1770 cm⁻¹ et 1710 cm⁻¹ du groupe phtalimide -CO-N-CO-
- Analyse élémentaire :: 3,53% d'azote correspondant à 2,52 mmoles de groupe -NH₂/g de résine.

### Exemple 2 Obtention d'une deuxième résine de formule (I) dans laquelle L représente -CH₂-.

(a) La résine PS-DVB déjà chlorométhylée a les caractéristiques suivantes, déterminées par analyse.
   - Teneur en chlore : 19,32% en poids (Cl = 5,44 méq/g de résine)
   - Surface spécifique : 22,5 m²/g
   - Diamètre moyen des pores : 20 Å
   - Volume des pores : 69%
(b) Fonctionnalisation de cette résine par le phtalimide de potassium.
   10 g (0,054 éq.Cl) de cette résine chlorométhylée sont mis dans une solution de 10,1 g (0,054 mole) de phtalimide de potassium dans 150 ml de diméthylformamide (DMF) anhydre à une température de 50°C, puis la suspension ainsi obtenue est laissée à cette température pendant 24 heures. Après retour à la température ambiante la résine est filtrée, lavée au DMF, au méthanol, à l'eau, puis de nouveau au méthanol et enfin à l'acétone. Après séchage sous vide, on obtient 15,4 g de résine.
(c) Formation de l'amine primaire par hydrazinolyse.
   La résine phtalimidométhylée ci-dessus (15,4 g) est mise dans une solution de 6,6 ml (0,136 mole) d'hydrate d'hydrazine dans 150 ml d'éthanol absolu après addition de 1 g de soude en pastilles et le tout est agité mécaniquement et porté au reflux pendant 48 heures. Puis on filtre la résine à chaud et on la lave à l'éthanol puis à l'eau et enfin on la traite par 400 ml d'une solution aqueuse de potasse à 10% en poids.
   Après ce traitement, la résine est lavée à l'eau jusqu'à neutralité, puis à l'éthanol et enfin à l'acétone. Après séchage sous vide, à 60°C, on obtient 10 g de résine.
   Analyse élémentaire : N = 5,46% en poids soit une capacité de 3,92 mmoles de fonction amine primaire (-NH₂)/g de résine.

### Exemple 3 Obtention d'une troisième résine de formule (I) dans laquelle L représente le radical -CH₂-O-CH₂-CH₂-

La résine PS-DVB chlorométhylée utilisée est la même que celle ci-dessus en 2.a).

### a) Obtention de la résine amine primaire.

On prépare une solution de 6,1 g d'hydrure de sodium à 60% (0,1525 mole) dissous dans 150 ml de THF anhydre (distillé sur sodium). A cette solution on additionne lentement sous atmosphère d'azote une solution de 9,8 ml (0,1633 mole) d'éthanolamine dans 100 ml de THF anhydre. Sous agitation, on maintient le milieu réactionnel à 20°C pendant 1 heure puis on le porte au reflux pendant 2 heures. Après refroidissement à 20°C, on introduit lentement 20 g de la résine chlorométhylée. Toujours sous agitation on porte le milieu réactionnel à 70°C et on le maintient à cette température pendant 48 heures. Après refroidissement, on filtre la résine puis on la lave successivement à l'eau, avec une solution aqueuse à 5% en poids de potasse, puis à l'eau jusqu'à neutralité et enfin au méthanol.
On sèche la résine sous vide, à 60°C et on obtient 20,1 g de la résine amine primaire portant le bras espaceur -CH₂-O-CH₂-CH₂-.
Analyse élémentaire: N = 4,28% en poids, soit une capacité de 3,05 mmoles de fonction amine primaire (-NH₂) /g de résine.

### B) Préparation de di, n-butyldisulfure par réaction du n-butylmercaptan avec le soufre en présence de résines à fonction amine.

Les essais de préparation du di, n-butyldisulfure suivants ont été réalisés dans des conditions expérimentales identiques en changeant seulement de résines PS-DVB mais en utilisant pour chaque essai une quantité de résine sèche correspondant au même nombre d'équivalents basiques de résines.

Un essai comparatif a été effectué avec la résine Amberlyst A-21 sèche à fonction amine tertiaire.

Ces essais sont effectués chacun dans un réacteur constitué d'un erlenmeyer en verre d'un volume de 50 ml, à deux tubulures, comportant un réfrigérant à reflux refroidi par une circulation d'eau et une gaine thermométrique pour la mesure de la température du milieu réactionnel. Ce réacteur est chauffé au moyen d'un bain d'huile placé sur la plaque d'un agitateur magnétique chauffant, et l'agitation est effectuée à l'aide d'un aimant Téflonné placé dans le milieu réactionnel.

On introduit dans le réacteur 26,58 g (0,295 mole) de n-butylmercaptan avec 4,5 g (0,147 mole) de soufre finement broyé et la résine en une quantité correspondant à 0,41 à 0,44 méq de fonction -NH₂, puis sous agitation on porte le milieu à 60°C. Après disparition totale du soufre solide (généralement au bout de 15 min.) on prélève des échantillons à des temps déterminés et on les analyse par chromatographie en phase gazeuse, sur une colonne capillaire Hewlett-Packard Ultra-1 de 50 m de long, pour en déterminer la teneur en di, n-butyldisulfure.
Les résultats figurent dans le Tableau I suivant.

La quantité en poids de di, n-butyldisulfure formé est noté S₂% en fonction du temps en min.

**Tableau I**

| Référence Résine | (A-21)* | A-107 | A-109 | Exemple N°1 (-NH₂) | Exemple N°3 (-NH₂) |
|---|---|---|---|---|---|
| Nature | Macroporeuse | Macroporeuse | Macroporeuse | Macroporeuse | Macroporeuse |
| Fonctionnalité | -CH₂-N(CH₃)₂ | -CH₂-NH₂ | -CH₂-NH₂ | -CH₂-NH₂ | -CH₂-O-CH₂-CH₂-NH₂ |
| (mmole/g) | 4,4 | 4,1 | 4,3 | 2,52 | 3,05 |
| Poids de résine | 0,1 g | 0,1 g | 0,1 g | 0,17 g | 0,15 g |
| Temps (min.) | "S₂"% | "S₂"% | "S₂"% | "S₂"% | "S₂"% |
| 30 | 54,6 | 60,7 | 64,9 | 56,1 | 50,9 |
| 60 | 58,9 | 67,0 | 74,2 | 62,9 | 66,3 |
| 90 | 65,0 | 76,7 | 81,2 | 70,1 | 74,0 |
| 180 | 72,0 | 82,1 | 84,9 | 78,1 | 82,5 |
| 360 | 79,5 | 84,6 | 85,1 | 82,5 | 85,3 |

| | | | | | |
|---|---|---|---|---|---|
| * essai comparatif | | | | | |

Ces résultats montrent que les résines macroporeuses à fonction amine primaire ont une activité catalytique supérieure à celle de l'essai comparatif.

### C) Préparation de di, tert-butylpolysulfures par réaction du tert-butylmercaptan avec le soufre en présence de résines à fonction amine.

Ces essais sont réalisés dans le même appareillage que précédemment en B).

On introduit 26,5 g (0,294 mole) de tert-butylmercaptan avec 18 g (0,56 mole) de soufre, finement broyé puis de 0,6 à 0,8 g de résine en une quantité correspondant à 2,4 à 2,6 méq d'amine.

On porte ensuite le milieu sous agitation, à 60°C. On note la durée pour obtenir la disparition totale du soufre solide. Après 90 min. de réaction, on effectue un premier prélèvement du milieu réactionnel liquide, suivi d'autres prélèvements successifs au cours de la réaction. On analyse les échantillons prélevés par chromatographie en phase gazeuse sur une colonne capillaire Hewlett-Packard Ultra-1 de 50 m de long.

Les résultats figurent dans le Tableau II suivant :
on a noté pour chaque résine testée le temps au bout duquel tout le soufre solide a disparu par dissolution et également la teneur en tert-butylmercaptan (TBM % en poids) restant dans le milieu réactionnel.

**Tableau II**

| Référence Résine | (A-21) * | A-107 | A-109 | Exemple N°3 (-NH₂) |
|---|---|---|---|---|
| Nature | [Macroporeuse] | Macroporeuse | Macroporeuse | Macroporeuse |
| Fonctionnalité | -CH₂-N(CH₃)₂ | -CH₂-NH₂ | -CH₂-NH₂ | -CH₂-O-CH₂-CH₂-NH₂ |
| (mmole/g) | 4,4 | 4,1 | 4,3 | 3,05 |
| Poids de résine | 0,6 g | 0,6 g | 0,6 g | 0,8 g |
| Dissolution du soufre | 80 min. | 90 min. | 60 min. | 80 min. |
| Temps (min.) | TBM % | TBM % | TBM % | TBM % |
| 90 | 4,1 | 6,9 | 6,6 | 3,5 |
| 180 | 3,4 | 4,1 | 3,4 | 3,2 |

| | | | | |
|---|---|---|---|---|
| * essai comparatif | | | | |

On note que la résine de l'Exemple N°3 provoque une disparition plus rapide du TBM que la résine de l'essai comparatif.

### D) Préparation de di, tert-butyltrisulfure par rétrogradation par le tert-butylmercaptan de di, tert-butylpolysulfures de rangs élevés en soufre.

Le di, tert-butylpolysulfure utilisé a une masse moléculaire moyenne de 250 (teneur en soufre de 54,4%), et une teneur en di, tert-butyltrisulfure de 29,5%, le complément à 100% en poids étant constitué de polysulfures (Sₓ avec x > 3) de rangs plus élevés en soufre.

Les essais sont réalisés dans le même appareillage que précédemment décrit en B) et C).

On introduit dans le réacteur 10 g (0,0365 mole) de di, tert-butylpolysulfure avec 19,71 g (0,219 mole) de tert-butylmercaptan ainsi que la résine choisie en une quantité correspondant à 2 à 2,2 méq d'amine, puis sous agitation on porte le milieu réactionnel à 60°C. A intervalles de temps déterminés on prélève des échantillons et on les analyse par chromatographie en phase gazeuse sur une colonne capillaire Hewlett-Packard Ultra-1 de 50 m de long.

Le suivi chromatographique permet de déterminer la teneur en di, tert-butyltrisulfure formé au cours du temps.

Les résultats figurent dans le Tableau III suivant :
on note la proportion de di, tert-butyltrisulfure (S₃%) formé en fonction du temps en min.

**Tableau III**

| Référence Résine | (A-21) * | A-107 | A-109 | Exemple N°3 (-NH₂) |
|---|---|---|---|---|
| Nature | Macroporeuse | Macroporeuse | Macroporeuse | Macroporeuse |
| Fonctionnalité | -CH₂-N(CH₃)₂ | -CH₂-NH₂ | -CH₂-NH₂ | -CH₂-O-CH₂-CH₂-NH₂ |
| (mmole/g) | 4,4 | 4,1 | 4,3 | 3,05 |
| Poids de résine | 0,5 g | 0,5 g | 0,5 g | 0,7 g |
| Temps (min.) | "S₃"% | "S₃"% | "S₃"% | "S₃"% |
| 30 | 50,9 | 41,6 | 44,5 | 40,9 |
| 60 | 58,5 | 51,4 | 58,6 | 50,8 |
| 120 | 65,2 | 60,5 | 78,9 | 60,1 |
| 180 | 68,8 | 69,6 | 87,8 | 69,1 |
| 240 | 70,9 | 73,1 | 90,4 | 72,1 |
| 300 | 72,8 | 78,9 | 93,8 | 75,6 |

| | | | | |
|---|---|---|---|---|
| * essai comparatif | | | | |

On remarque ici que la résine A-109 est la plus active des quatre et que les trois résines à fonction -NH₂ sont plus actives que celle de l'essai comparatif.

## Revendications

1. Procédé de préparation de disulfures et polysulfures organiques par réaction du soufre sur un mercaptan ou sur un polysulfure de rang inférieur en soufre pour le transformer en polysulfure de rang supérieur, ou encore par réaction d'un mercaptan sur un polysulfure organique de rang élevé en soufre pour le transformer en polysulfure de rang inférieur en soufre, en présence d'un catalyseur sous forme de résine à fonction basique, caractérisé en ce que la résine est à base de polystyrène-divinylbenzène (PS-DVB) fonctionnalisée par des groupes amine primaire et ayant la formule générale (I) : 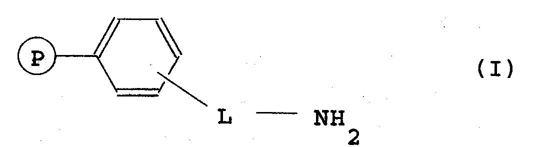 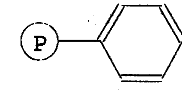 étant le support résine PS-DVB,
L étant un radical organique linéaire ayant une longueur égale ou supérieure à celle du radical méthylène (-CH₂-).

2. Procédé suivant la revendication 1, caractérisé en ce que la résine de formule générale (I) est macroréticulée et de structure macroporeuse.

3. Procédé suivant la revendication 1 ou 2, caractérisé en ce que L représente un méthylène (-CH₂-).

4. Procédé suivant la revendication 1 ou 2, caractérisé en ce que L représente un radical de formule (II)
-CH₂-(-X-CH₂-CH₂-)ₘ- (II)
X représentant l'oxygène (-O-) ou le soufre (-S-) et m valant 1 ou 2.

5. Procédé suivant la revendication 4, caractérisé en ce que X représente l'oxygène et que m vaut 1.

6. Procédé suivant la revendication 4, caractérisé en ce que X représente le soufre et que m vaut 1.

7. Procédé suivant l'une des revendications 1 à 6, caractérisé en ce que les disulfures, polysulfures organiques et mercaptans ont des radicaux hydrocarbonés R choisis parmi un groupe alkyle, cycloalkyle, aryle, aralkyle, et alkylaryle.

8. Procédé suivant la revendication 7, caractérisé en ce que le radical R porte un ou plusieurs groupes fonctionnels.

9. Procédé suivant la revendication 6, caractérisé en ce que la résine est présente en une quantité allant de 0,01 à 20 parties en poids pour 100 parties en poids de mélange réactionnel, résine comprise.

10. Procédé suivant l'une des revendications 1 à 9, caractérisé en ce que la réaction est effectuée à une température de - 10°C à 150°C.

11. Procédé suivant la revendication 10, caractérisé en ce que la température est de + 10°C à 120°C.

## Patentansprüche

1. Verfahren zur Herstellung von organischen Di- und Polysulfiden durch Einwirkung von Schwefel auf ein Mercaptan oder ein Polysulfid mit niedrigem Schwefelgehalt, um es in ein Polysulfid mit höherem Schwefelgehalt umzuwandeln, oder durch Einwirkung eines Mercaptans auf ein organisches Polysulfid mit hohem Schwefelgehalt, um es in ein Polysulfid mit niedrigem Schwefelgehalt umzuwandeln, in Gegenwart eines Katalysators in Form eines basischen Harzes, dadurch gekennzeichnet, daß das Harz auf der Grundlage von Polystyrol-Divinylbenzol (PS-DVB) durch primäre Aminogruppen der allgemeinen Formel (I) funktionell wirksam gemacht ist: 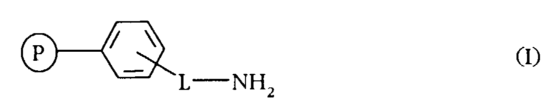 wobei 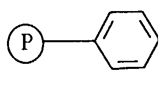 das Trägerharz PSB-DVB und
L ein organischer linearer Rest mit einer Länge gleich oder größer der Länge des Methylenrestes (-CH₂-) ist.

2. Verfahren nach Anspruch 1, dadurch gekennzeichnet, daß das Harz der allgemeinen Formel (I) makrovernetzt ist und eine makroporöse Struktur hat.

3. Verfahren nach Anspruch 1 oder 2, dadurch gekennzeichnet, daß L Methylen (-CH₂-) bedeutet.

4. Verfahren nach Anspruch 1 oder 2, dadurch gekennzeichnet, daß L einen Rest der Formel (II)
-CH₂-(-X-CH₂-CH₂-)ₘ- (II)
bedeutet, in der X Sauerstoff (-O-) oder Schwefel (-S-) bedeutet und m = 1 oder 2 ist.

5. Verfahren nach Anspruch 4, dadurch gekennzeichnet, daß X Sauerstoff bedeutet und daß m = 1 ist.

6. Verfahren nach Anspruch 4, dadurch gekennzeichnet, daß X Schwefel bedeutet und daß m = 1 ist.

7. Verfahren nach einem der Ansprüche 1 bis 6, dadurch gekennzeichnet, daß die organischen Di- und Polysulfide und Mercaptan Wasserstoffreste R haben, die aus einer Alkyl-, Cycloalkyl-, Aryl-, Aralkyl- und Alkylarylgruppe gewählt sind.

8. Verfahren nach Anspruch 7, dadurch gekennzeichnet, daß der Rest R eine oder mehrere funktionelle Gruppen trägt.

9. Verfahren nach Anspruch 6, dadurch gekennzeichnet, daß das Harz in einer Menge von 0,01 bis 20 Gew.-Teilen, bezogen auf 100 Gew.-Teile des Reaktionsgemisches einschließlich des Harzes, vorliegt.

10. Verfahren nach einem der Ansprüche 1 bis 9, dadurch gekennzeichnet, daß die Reaktion bei einer Temperatur von -10 °C bis 150 °C erfolgt.

11. Verfahren nach Anspruch 10, dadurch gekennzeichnet, daß die Temperatur zwischen +10 °C und 120 °C liegt.

## Claims

1. Process for the preparation of organic disulphides and polysulphides by reaction of sulphur with a mercaptan or with a polysulphide of lower sulphur order in order to convert it into polysulphide of higher order, or alternatively by reaction of a mercaptan with an organic polysulphide of high sulphur order in order to convert it into polysulphide of lower sulphur order, in the presence of a catalyst in the form of a resin with a basic function, characterized in that the resin is based on polystyrenedivinylbenzene (PS-DVB) functionalized with primary amine groups and having the general formula (I): 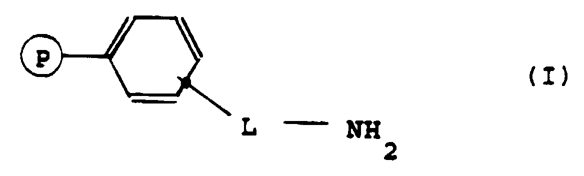 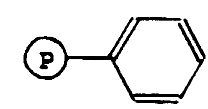 being the PS-DVB resin support,
L being a linear organic radical which is as long as or longer than the methylene radical (-CH₂-).

2. Process according to Claim 1, characterized in that the resin of general formula (I) is macrocrosslinked and of macroporous structure.

3. Process according to Claim 1 or 2, characterized in that L represents a methylene (-CH₂-).

4. Process according to Claim 1 or 2, characterized in that L represents a radical of formula (II)
-CH₂-(-X-CH₂-CH₂-)ₘ- (II)
X representing oxygen (-O-) or sulphur (-S-) and m being equal to 1 or 2.

5. Process according to Claim 4, characterized in that X represents oxygen and m is equal to 1.

6. Process according to Claim 4, characterized in that X represents sulphur and m is equal to 1.

7. Process according to one of Claims 1 to 6, characterized in that the mercaptans and organic disulphides and polysulphides have hydrocarbon radicals R chosen from an alkyl, cycloalkyl, aryl, aralkyl and alkylaryl group.

8. Process according to Claim 7, characterized in that the radical R bears one or more functional groups.

9. Process according to Claim 6, characterized in that the resin is present in an amount ranging from 0.01 to 20 parts by weight per 100 parts by weight of reaction mixture, resin included.

10. Process according to one of Claims 1 to 9, characterized in that the reaction is carried out at a temperature of from -10°C to 150°C.

11. Process according to Claim 10, characterized in that the temperature is from +10°C to 120°C.
